# DEMANDE DE BREVET EUROPEEN

(11) **EP 0 931 556 A1**
(43) Date de publication de la demande: **28.07.1999**
(21) Numéro de dépôt: 98490006.8
(22) Date de dépôt: 23.01.1998
(51) Int. Cl.: A61M 5/32

(54) **Récipient de collecte des aiguilles souillées**

(71) Demandeur: Ranson, Marie Noelle, 59500 Douai (FR)
(72) Inventeur: Ranson, Marie Noelle, 59500 Douai (FR)
(74) Mandataire: Ecrepont, Robert

(57) **Abrégé**

L'invention se rapporte à un récipient (1) de collecte des aiguilles souillées délimité par une enveloppe (3) présentant un orifice (4) d'introduction d'une aiguille, cette aiguille étant emboitée sur un embout (6) présenté par le corps d'une seringue (5).

Il est caractérisé en ce qu'il comprend :
- au moins une lame (7) se déplacant dans un plan sécant (8) à l'axe (9) matérialisé par l'embout (6) prolongé par l'aiguille (2) lorsque ceux- ci sont introduits au travers de l'orifice (4) d'introduction et
- des moyens (10) de manoeuvre de la lame depuis une position dite écartée, où cette lame (1O) est éloignée de l'axe précité pour permettre l'introduction de l'embout prolongé par l'aiguille, vers une position active où cette lame (7) sectionne l'embout (6) de la seringue.

## Description

L'invention se rapporte à un récipient de collecte des aiguilles souillées.

Pour éviter que le personnel médical ne se blesse en replaçant le capuchon protecteur sur l'aiguille souillée emboitée sur l'embout que présente l'extrémité du corps de la seringue, il est recommandé de ne plus remettre en place ledit capuchon.

Il est donc nécessaire de se débarrasser au moins de l'aiguille dans des conditions assurant la sécurité de tous.

On connait à ce jour des récipients pourvus d'un orifice permettant de collecter la seringue pourvue encore, à son extrémité, de l'aiguille souillée.

Ces récipients sont de grandes tailles et ne sont pas d'un emploi commode pour le personnel médical procurant des soins à domicile.

Pour remédier à cet inconvénient, on connait des récipients collecteurs de plus petite taille présentant, de part et d'autre de l'orifice d'introduction de l'aiguille, des mâchoires fixes ou actionnées par un moyen de manoeuvre déplaçant les dites mâchoires entre une position dite écartée permettant d'engager l'aiguille au travers de l'orifice et une position dite rapprochée où les dites mâchoires sont suffisamment prôches l'une de l'autre pour saisir et maintenir l'aiguille le temps nécessaire pour la déboiter de l'embout présenté par la seringue.

Lorsque la seringue est séparée de l'aiguille, l'utilisateur manoeuvre de nouveau les mâchoires pour les amener à nouveau vers la position écartée de sorte que l'aiguille tombe par gravité dans le récipient.

La seringue peut alors être placée dans une poubelle du type à usage domestique sans qu'il y ait un risque de se piquer.

Les seringues bien que souillées pourraient toutefois être réutilisées par des gens mal intentionnés.

Pour éviter cela, le piston des seringues présente une amorce de rupture permettant à l'utilisateur de le rompre et de rendre la seringue inutilisable.

Malheureusement, malgré cette amorce de rupture, il est très difficile de le rendre inutilisable.

Un des résultats que l'invention vise à obtenir est notamment de remédier aux inconvénients précités et, à cet effet, l'invention a pour objet un récipient de collecte des aiguilles usagées délimité par une enveloppe présentant un orifice d'introduction de l'aiguille portée par un embout que présente le corps de la seringue, ce récipient de collecte étant caractérisé en ce qu'il comprend :
- au moins une lame se déplacant dans un plan sécant à l'axe matérialisé par l'embout prolongé par l'aiguille lorsque ceux- ci sont introduits au travers de l'orifice d'introduction et
- des moyens de manoeuvre de la lame depuis une position dite écartée, où cette lame est éloignée de l'axe précité pour permettre l'introduction de l'embout prolongé par l'aiguille, vers une position active où cette lame sectionne l'embout de la seringue.

L'invention sera bien comprise à l'aide de la description ci-aprés faite à titre d'exemple non limitatif en regard du dessin ci-annexé qui représente schématiquement :
- figure 1 : une vue en coupe d'un récipient de collecte selon F,
- figure 2 : un détail du récipient,
- figures 3 et 4 : des variantes de réalisation.

En se reportant au dessin, on voit :
- d'une part, un récipient 1 de collecte des aiguilles 2 usagées délimité par une enveloppe 3 présentant un orifice 4 d'introduction d'une aiguille et,
- d'autre part, une seringue 5 comprenant un corps tubulaire présentant à l'une de ses extrémités un embout 6 sur lequel s'emboite l'une des extrémités de l'aiguille 2.

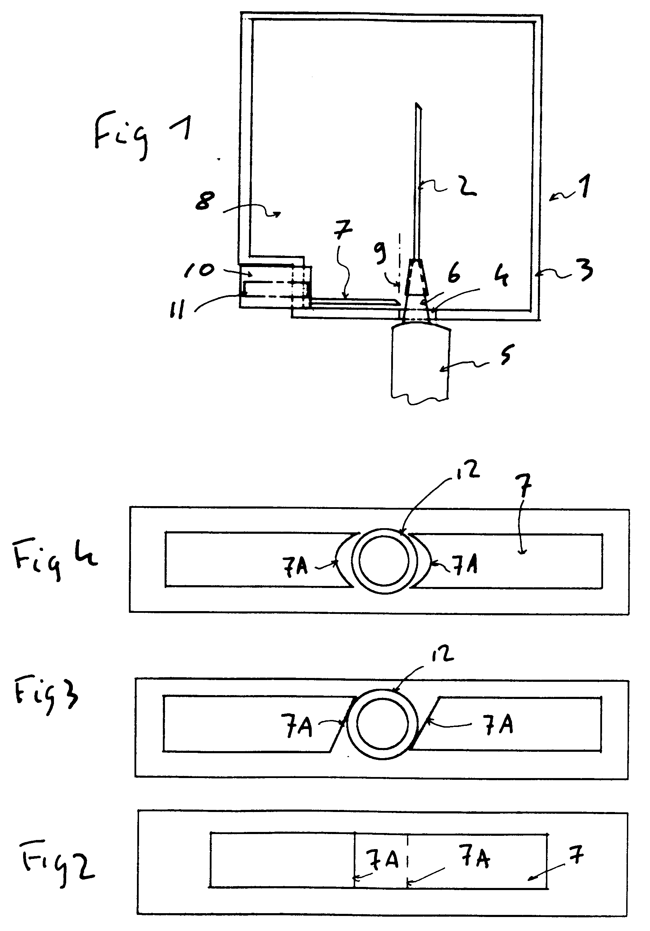

Selon une caractéristique de l'invention, le récipient comprend :
- au moins une lame 7 se déplacant dans un plan sécant 8 à l'axe 9 matérialisé par l'embout 6 prolongé par l'aiguille 2 lorsque ceux- ci sont introduits au travers de l'orifice 4 d'introduction et
- des moyens 10 de manoeuvre de la lame depuis une position dite écartée (figures 3, 4) où cette lame 10 est éloignée de l'axe précité pour permettre l'introduction de l'embout prolongé par l'aiguille vers une position active (figure 2) où cette lame 7 sectionne l'embout 6 de la seringue.

La lame 7 en agissant comme une guillotine sectionne l'embout de sorte que l'aiguille encore emboitée sur l'embout tombe au fond du récipient et rend inutilisable le corps de la seringue.

Par ailleurs, cette lame 7 est, par un moyen 11 de rappel, ramenée constamment vers la position active dans laquelle elle obture l'orifice 4 d'introduction.

Un joint d'étanchéité 12 placé autour de l'orifice 4 et entre la lame et la face interne de l'enveloppe évite toutes fuites de liquide.

La lame forme ainsi un clapet.

De préférence, le récipient comprend deux lames 7 (figures 2, 3, 4) qui, situées dans des plans parallèles, sont par un moyen de manoeuvre approprié déplacées dans des directions antagonistes.

L'extrémité de la lame est bisautée.

Le bord 7A tranchant est :
- soit perpendiculaire (figure 2) à l'axe de déplacement de la lame,
- soit oblique (figure 3) à la manière d'une guillotine ou
- encore courbe (figure 4) à la manière d'un coupe-cigare.

L'enveloppe du récipient collecteur sera par exemple en polypropylène rigide.

## Revendications

1. Récipient (1) de collecte des aiguilles souillées délimité par une enveloppe (3) présentant un orifice (4) d'introduction d'une aiguille, cette aiguille étant emboitée sur un embout (6) présenté par le corps d'une seringue (5),
ce récipient étant **CARACTERISE** en ce qu'il comprend :
- au moins une lame (7) se déplacant dans un plan sécant (8) à l'axe (9) matérialisé par l'embout (6) prolongé par l'aiguille (2) lorsque ceux- ci sont introduits au travers de l'orifice (4) d'introduction et
- des moyens (10) de manoeuvre de la lame depuis une position dite écartée, où cette lame (1O) est éloignée de l'axe précité pour permettre l'introduction de l'embout prolongé par l'aiguille, vers une position active où cette lame (7) sectionne l'embout (6) de la seringue.

2. Récipient selon la revendication 1 **caractérisé** en ce que la lame (7) est, par un moyen (11) de rappel, ramenée constamment ves la position active dans laquelle elle obture l'orifice (4) d'introduction.

3. Récipient selon la revendication 2 **caractérisé** en ce qu'un joint d'étanchéité (12) placé autour de l'orifice (4) et entre la lame et la face interne de l'enveloppe évite toutes fuites de liquide.

4. Récipient selon la revendication 2 **caractérisé** en ce que la lame forme un clapet.

5. Récipient selon l'une quelconque des revendications 1 à 4 **caractérisé** en ce qu'il comprend deux lames (7) qui, situées dans des plans parallèles, sont par un moyen de manoeuvre approprié déplacées dans des directions antagonistes.

6. Récipient selon l'une quelconque des revendications 1 à 5 **caractérisé** en ce que le bord (7A) tranchant est perpendiculaire à l'axe de déplacement de la lame.

7. Récipient selon l'une quelconque des revendications 1 à 5 **caractérisé** en ce que le bord (7A) tranchant est oblique à la manière d'une guillotine.

8. Récipient selon l'une quelconque des revendications 1 à 5 **caractérisé** en ce que le bord (7A) tranchant est courbe à la manière d'un coupe-cigare.

9. Récipient selon la revendication 1 **caractérisé** en ce que l'enveloppe du récipient collecteur est en polypropylène rigide.
